Europäisches Patentamt

European Patent Office

Office européen des brevets

(19)

(11) Veröffentlichungsnummer: **0 026 291**
**B1**

(12) # EUROPÄISCHE PATENTSCHRIFT

(45) Veröffentlichungstag der Patentschrift:
09.02.83

(51) Int. Cl.³: **C 07 C 47/544, C 07 C 45/42**

(21) Anmeldenummer: **80104456.1**

(22) Anmeldetag: **29.07.80**

(54) Verfahren zur Herstellung von Terephthalaldehyd bzw. Isophthalaldehyd.

(30) Priorität: **28.08.79 DE 2934614**

(43) Veröffentlichungstag der Anmeldung:
**08.04.81 Patentblatt 81/14**

(45) Bekanntmachung des Hinweises auf die Patenterteilung:
**09.02.83 Patentblatt 83/6**

(84) Benannte Vertragsstaaten:
**AT BE CH DE FR GB IT LI NL SE**

(56) Entgegenhaltungen:
**EP-A-0 003 230**
**Chemical Abstracts, Band 63, Nr. 2, 1965, Colum-**
**bus, Ohio, USA, L. V. SHUBINA et al., »Synthesis**
**of 1.2- and 1.3-distyrylbenzenes«, column 1718cd**

(73) Patentinhaber: **DYNAMIT NOBEL**
**AKTIENGESELLSCHAFT, Patentabteilung**
**Postfach 1209, D-5210 Troisdorf, Bez. Köln (DE)**

(72) Erfinder: **Englaender, Fritz, Dr., Flossweg 10,**
**D-5300 Bonn 2 (DE)**
Erfinder: **Steffen, Klaus-Dieter, Dr., Röchelstrasse 36,**
**D-5202 Hennef 1 (DE)**

### Verfahren zur Herstellung von Terephthalaldehyd bzw. Isophthalaldehyd

Die Erfindung bezieht sich auf ein Verfahren zur Herstellung von Terephthalaldehyd bzw. Isophthalaldehyd.

Zur Vereinfachung werden folgende Abkürzungen eingeführt:

p-100 = p-Methylbenzylchlorid
p-200 = p-Methylbenzalchlorid
p-101 = 1,4-Bis-(chlormethyl-)benzol
p-201 = 1-Dichlormethyl-4-chlormethyl-benzol
p-202 = 1,4-Bis-(dichlormethyl-)benzol
p-302 = 1-Trichlormethyl-4-dichlor-methyl-benzol

Die Herstellung einer Anzahl von Aldehyden kann durch eine Reaktion der entsprechenden $-CH_2Cl$-Verbindungen mit Hexamethylentetramin erfolgen, während andere Aldehyde offenbar auf diesem Wege nicht zugängig sind.

Ein möglicher Weg zur Herstellung des Terephthalaldehyds besteht in der Umsetzung des reinen 1,4-Bis(chlormethyl-)benzols mit Hexamethylentetramin führt aber nur zu Ausbeuten von 34% der Theorie.

Dieser Weg weist verschiedene weitere Nachteile auf: Die Seitenkettenchlorierung des p-Xylols verläuft nicht selektiv, vielmehr erhält man die möglichen Chlorierungsstufen nebeneinander. Das Verhältnis der einzelnen Chlorierungsprodukte hängt ausschließlich vom Chlorierungsgrad ab. Die gebildete Menge 1,4-Bis-(chlormethyl-)benzol durchläuft während der Chlorierung ein Maximum, das bei 46 Gew.-% Cl und einem Chlorierungsgrad von 2,4 liegt. Der größte Anteil p-101 liegt also vor, wenn der Chlorierungsgrad den Wert 2,0 für reines p-101 bereits weit überschritten hat. Bei weiterer Chlorierung nimmt 1,4-Bis-(chlormethyl-)benzol wieder ab zugunsten der Bildung von z. B. 1,4-Bis-(dichlormethyl-)benzol und noch höher chlorierter Xylole.

Demgemäß ist die Abtrennung des 1,4-Bis-(chlormethyl-)benzols aus dem 6-Komponentengemisch aufwendig, zumal die Siedepunkte nahe zusammenliegen.

Es wurde nun gefunden, daß man diese Nachteile dadurch erheblich verringern kann, daß man Gemische der Verbindungen, die bei der Chlorierung von p-Xylol bzw. m-Xylol entsteht, ohne weitere Trennung für die Umsetzung zu Terephthalaldehyd einsetzt.

Gegenstand der Erfindung ist ein Verfahren zur Herstellung von Terephthalaldehyd aus p-Xylol oder von Isophthalaldehyd aus m-Xylol aus Seitenkettenchlorierungsprodukten der jeweiligen Xylole durch Hydrolyse, welches dadurch gekennzeichnet ist, daß das jeweilige Xylol zu einem Gemisch von Chlorierungsprodukten mit einem Chlorierungsgrad von 2,5 bis 3,5 durch radikalische Chlorierung der Seitenkette chloriert, darauf dieses Gemisch von jeweiligen Chlorierungsprodukten mit Hexamethylentetramin in wäßriger Lösung bei Temperaturen von 70 bis 140°C umgesetzt, und das jeweilige Aldehyd anschließend an die Hydrolyse gewonnen wird.

Der Chlorierungsgrad soll bevorzugt 2,6 bis 3,1 betragen. Die Hexamethylentetraminmenge kann in stöchiometrischen Anteilen oder im Überschuß eingesetzt werden, wobei stöchiometrische Mengen bedeuten, daß pro Mol $-CH_2Cl$-Gruppe ein Mol Hexamethylentetramin zur Anwendung gelangt. Mit steigendem Überschuß an Hexamethylentetramin nimmt die Aldehydausbeute zu. Überschüsse von mehr als 30% steigern jedoch nicht mehr die Wirtschaftlichkeit. Der Überschuß an Hexamethylentetramin beträgt daher allgemein 0 bis 30 Gew.-%, vorzugsweise 10 bis 20 Gew.-%.

Anstelle von Hexamethylentetramin kann man auch eine wäßrige Lösung von Ammoniak und Formaldehyd einsetzen, womit man offensichtlich wegen des chemischen Gleichgewichts in wäßriger Lösung zwischen Hexamethylentetramin und Ammoniak-Formaldehyd ähnliche Ergebnisse erzielt. Das Mengenverhältnis von Ammoniak und Formaldehyd soll in dieser Lösung allgemein das Molverhältnis 4 : 6 des Hexamethylentetramins einhalten, kann jedoch um bis zu 10% nach beiden Seiten abweichen.

Nach der Hydrolyse kann das Produkt durch Abfiltrieren und Auswaschen der wasserlöslichen Salze gewonnen werden. Die Reinigung erfolgt durch Destillation oder ggf. Kristallisation bzw. Sublimation.

Die Umsetzung des Chlorxylol-Gemisches mit Hexamethylentetramin findet vorzugsweise bei Temperaturen von 100 bis 115°C statt.

Überraschenderweise sind die Aldehydausbeuten bei Einsatz des Chlorierungsgemisches wesentlich höher, als wenn man von reinem p-101 ausgeht.

Die Ausbeuten an Aldehyd betragen bis zu 77%, bezogen auf das eingesetzte p-Xylol bzw. m-Xylol.

Das vorliegende Verfahren erlaubt also eine vereinfachte Synthese der nicht leicht zugänglichen Stoffe Terephthalaldehyd bzw. Isophthalaldehyd bei stark verbesserter Gesamtausbeute.

Das Chlorierungsgemisch wird durch radikalische Chlorierung von Xylol bereitet. Es kann mit Lösungsmittel wie z. B. $CCl_4$ oder auch ohne Lösemittel gearbeitet werden. Die Reaktionstemperatur von 50 bis 150°C, vorzugsweise 80 bis 90°C, wird so eingestellt, daß das Reaktionsgemisch geschmolzen vorliegt. Die für die Reaktion benötigten Radikale können sowohl durch Bestrahlung mit UV-Licht als auch durch Zusatz von Radikalbildnern, z. B. Azo-bis-diisobutyronitril, erzeugt werden.

Die Chlorierung ist selbstverständlich so auszuführen, daß im Kern chlorierte Produkte nur in sehr geringer Menge entstehen. Der Chlorierungsgrad der Chlorierungsgemische ist in bekannter Weise zu errechnen, indem der Chlorierungsgrad der enthaltenen Stoffe, d. h. z. B. 1 für p-(bzw. m-)100 oder 2 für p-101 und p-200, mit dem analytisch ermittelten Anteil dieser Stoffe in Gew.-% im Chlorierungsgemisch multipliziert, die erhaltenen Werte addiert und durch die Summe der Anteile dividiert werden.

Im Chlorierungsgemisch sollen p-100 und p-203 in Mengen von nicht mehr als 1% zugegen sein, da sie zu Ausbeuteverlusten führen. Aus p-100 bildet sich bei der Reaktion p-Toluylaldehyd und aus p-203 Terephthalaldehydsäure. Die hergestellten Dialdehyde können in bekannter Weise für zahlreiche Zwecke verwendet werden.

Insbesondere Terephthalaldehyd findet vielfältige Verwendung als Zwischenprodukt für organische Synthesen wie Herstellung optischer Aufheller und Farbstoffe, Pharmazeutika und Verbindungen gegen Viren, Bakterien, Pilze und Insekten.

### Beispiel 1

### Chlorierung von p-Xylol

p-Xylol (100 g = 0,943 Mol) werden bei einer Temperatur von 80 bis 90°C unter Bestrahlung mit einer UV-Lampe chloriert. Man erhält 189,7 g Chlorierungsgemisch der folgenden Produktverteilung (in Gew.-%): p-100: 0,3; p-200: 1,6; p-101: 32,9; p-102: 51,9; p-202: 12,1; p-203: 0,8.

### Umsetzung zum Aldehyd

189,7 g Chlorierungsgemisch (Chlorierungsgrad 2,79) 166,8 g Hexamethylentetramin und 630 g Wasser werden unter kräftigem Rühren 6 Stunden am Rückfluß erhitzt. Das Reaktionsgemisch wird gekühlt und der Aldehyd durch Abfiltrieren isoliert. Nach dem Trocknen erhält man ein leichtgelbgefärbtes Rohprodukt, das durch Destillation oder Sublimation weiter gereinigt werden kann.

Die Ausbeute an Reinprodukt beträgt 86,5 g entsprechend einer Ausbeute von 68,4%, bezogen auf eingesetztes p-Xylol. Der Aldehyd wies gaschromatographisch eine Reinheit von 98,7% auf.

### Beispiel 2

189,7 g Chlorierungsgemisch wie in Beispiel 1 werden mit 166,8 g Hexamethylentetramin und 630 g Wasser unter kräftigem Rühren 2 Stunden bei einer Temperatur von 110°C gehalten. Nach dem Abkühlen, Isolieren und Destillieren des Rohproduktes erhält man 88 g Terephthalaldehyd mit einer Reinheit von 98,1%, entsprechend einer Ausbeute von 69,6%, bezogen auf eingesetztes p-Xylol.

### Beispiel 3

Beispiel 2 wird wiederholt, jedoch mit 216,3 g Hexamethylentetramin entsprechend einem Überschuß von 30%.

Man erhält 97,7 g Terephthalaldehyd mit einer Reinheit von 97,8% entsprechend einer Ausbeute von 77,3%, bezogen auf eingesetztes p-Xylol.

### Beispiel 4

189,7 g Chlorierungsgemisch wie in Beispiel 1 werden zu einer Mischung von 612 g Formalinlösung 35%ig und 272 g Ammoniaklösung 29,8%ig gegeben. Das Gemisch wird 6 Stunden zum Rückfluß erhitzt und intensiv gerührt.

Nach der üblichen Aufarbeitung erhält man 82 g Terephthalaldehyd mit einer Reinheit von 98% entsprechend einer Ausbeute von 64,9%, bezogen auf eingesetztes p-Xylol.

### Beispiel 5

### Chlorierung von m-Xylol

m-Xylol (100 g = 0,943 Mol) werden bei einer Temperatur von 60−80°C unter Bestrahlung mit einer UV-Lampe chloriert. Man erhält 187,6 g Chlorierungsgemisch mit folgender Produktverteilung (Gew.-%): m-100: 2,0%; m-101: 46,0%; m-201: 39,2%; m-202: 11,2%.

### Umsetzung zum Aldehyd

187,6 g Chlorierungsgemisch (Chlorierungsgrad 2,61), 227 g Hexamethylentetramin und 625 g Wasser werden unter Rühren 2 Stunden auf 110°C erhitzt. Nach dem Abkühlen auf 100°C wird mit 30%iger Salzsäure bis zum pH 1 angesäuert und 10 Min. am Rückfluß erhitzt. Das Reaktionsgemisch wird gekühlt und der Aldehyd durch Abfiltrieren isoliert. Das nach dem Trocknen erhaltene Rohprodukt wird durch Destillation gereinigt. Die Ausbeute beträgt 83,8 g entsprechend 66,3%, bezogen auf eingesetztes m-Xylol.

### Beispiel 6

Durch Chlorierung von p-Xylol wird ein Chlorierungsgemisch mit einem Chlorierungsgrad von 3,01 und der Zusammensetzung (in Gew.-%): 100: 0,23; 200: 1,2; 101: 18,8; 201: 50,3; 301: 3,6; 202: 21,6; 302: 4,13; 303: 0,07 hergestellt.

170 kg dieses Chlorierungsgemisches, 134 kg Hexamethylentetramin und 510 l Wasser werden 2 Stunden unter Rühren auf 115°C erhitzt.

Nach Abfiltrieren des Rohprodukts und Destillation werden 66,4 kg Terephthalaldehyd, d. h. 61,1% der Theorie, bezogen auf p-Xylol, gewonnen.

## Patentansprüche

1. Verfahren zur Herstellung von Terephthalaldehyd aus p-Xylol oder von Isophthalaldehyd aus m-Xylol aus Seitenkettenchlorierungsprodukten der jeweiligen Xylole durch Hydrolyse, dadurch gekennzeichnet, daß das jeweilige Xylol zu einem Gemisch von Chlorierungsprodukten mit einem Chlorierungsgrad von 2,5 bis 3,5 durch radikalische Chlorierung der Seitenkette chloriert, darauf dieses Gemisch von jeweiligen Chlorierungsprodukten mit Hexamethylentetramin in wäßriger Lösung bei Temperaturen von 70 bis 140°C umgesetzt, und das jeweilige Aldehyd anschließend an die Hydrolyse gewonnen wird.

2. Verfahren nach Anspruch 1, dadurch gekennzeichnet, daß ein Überschuß an Hexamethylentetramin von 0 bis 30%, vorzugsweise 10 bis 20%, angewandt wird.

3. Verfahren nach mindestens einem der Ansprüche 1 oder 2, dadurch gekennzeichnet, daß anstelle des Hexamethylentetramins ein Gemisch von Formaldehyd und Ammoniak in Wasser eingesetzt wird.

## Claims

1. Process for the production of terephthalaldehyde from p-xylene or of isophthalaldehyde from m-xylene by hydrolysis of side chain chlorination products of the particular xylenes, characterised in that the particular xylene is chlorinated to a mixture of chlorination products with a degreee of chlorination of 2.5 to 3.5 by radical chlorination of the side chain, after which this mixture of particular chlorination products is reacted with hexamethylenetetramine in aqueous solution at temperatures of 70 to 140°C, and the particular aldehyde is subsequently recovered following the hydrolysis.

2. Process according to claim 1, characterised in that, an excess of hexamethylenetetramine of 0 to 30%, preferably 10 to 20%, is employed.

3. Process according to at least one of claims 1 or 2, characterised in that, in place of the hexamethylenetetramine, a mixture of formaldehyde and ammonia in water is employed.

## Revendications

1. Procédé de préparation, par hydrolyse, de téréphtalaldéhyde provenant du p-xylène ou d'isophtalaldéhyde provenant du m-xylène, à partir de produits de chloration des chaînes latérales des xylènes correspondants, caractérisé en ce que le xylène correspondant est chloré, par chloration radicalaire de la chaîne latérale, en un mélange de produits de chloration ayant un degré de chloration de 2,5 à 3,5, ce mélange des produits de chloration correspondants étant ensuite mis en réaction avec de l'hexaméthylènetétramine en solution aqueuse à des températures de 70 à 140°C, l'aldéhyde correspondant étant obtenu après l'hydrolyse.

2. Procédé selon la revendication 1, caractérisé en ce que l'on utilise un excès d'hexaméthylènetétramine de 0 à 30%, de préférence de 10 à 20%.

3. Procédé selon au moins l'une des revendications 1 ou 2, caractérisé en ce que l'on utilise à la place de l'hexaméthylènetétramine un mélange de formaldéhyde et d'ammoniac dans l'eau.